# EUROPEAN PATENT SPECIFICATION

(11) **EP 3 820 255 B1**
(45) Date of publication and mention of the grant of the patent: **28.02.2024**
(21) Application number: 19207141.3
(22) Date of filing: 05.11.2019
(51) Int. Cl.: H05B 47/115, A61N 5/06

(54) **SELF-CONFIGURING LIGHTING CONTROL**
SELBSTKONFIGURIERENDE BELEUCHTUNGSSTEUERUNG
COMMANDE D'ÉCLAIRAGE À CONFIGURATION AUTOMATIQUE

(43) Date of publication of application: 12.05.2021
(73) Proprietor: Helvar Oy Ab, 02150 Espoo (FI)
(72) Inventor: JUSLÉN, Henri, 02150 ESPOO (FI)
(74) Representative: Berggren Oy

(56) References cited:
- EP-A1- 2 919 562
- US-A1- 2013 221 858
- US-A1- 2014 266 669
- US-A1- 2016 091 217

## Description

### TECHNICAL FIELD

The example and non-limiting embodiments of the present invention relate to self-configuring and/or self-adaptive control of a lighting system.

### BACKGROUND

In prior art lighting systems, luminaires are controlled at least partially based on presence of one or more persons in the space illuminated by the luminaire such that the lights are switched and kept on while presence of one or more persons in the space is detected and switched and kept off while no persons are detected in the space. In a typical solution, a control device receives sensor signals from one or more passive infrared (PIR) sensors, determines occupancy or non-occupancy accordingly, and switches or keeps the lights on (at a normal light output level) in response to occupancy and switches/keeps the lights off (or at another low light output level) in response to non-occupancy. This kind of solution on one hand enables activating the light output of the luminaire such that light is provided in the space when it is needed, while on the other hand enables energy conservation via reducing the light output of the luminaire when no one is in the space.

In the prior art solutions wired or wireless centrally controlled lighting systems have been presented, where sensor signals are received by a central controller, which then sends control commands to individual luminaires, using, for example, DALI or some other lighting protocol.

Also, systems consisting of independent luminaires are known, where the sensor signals are received directly by a luminaire or luminaires adjacent to the sensor, and where a controller in the luminaires controls the light in accordance with the received sensor signals. In advanced systems, the luminaires may be able to send and receive information and be capable of adjusting their behavior or even control logic based on the information received from other luminaires and/or sensors.

It is also known that the circadian rhythm in humans and other beings is strongly affected by light. Therefore, it is important that the lighting does not disturb this circadian rhythm. Some prior art lighting solutions have been presented which can be used to support the circadian rhythm. In these kind of prior art solutions such supporting functionality is programmed into a control unit of a centrally controlled lighting system. This programming typically requires an expert and therefore it tends not to be updated. Thus, the programming and operation of the lighting system may not correspond to the circadian rhythm of the people actually influenced by the light.

There are also some prior art systems which are calculating what kind of individual needs of different people have for supporting their personal circadian rhythm. These kind of prior art solutions require wearables, applications and sophisticated personal lighting control which is not possible at e.g. at normal office or school environment.

One prior art solution is presented in US2014266669 which relates to devices that communicate with each other and/or with a central server or a cloud-computing system to provide home security objectives. In some embodiments US2014266669 discloses an "occupancy emulator" to emulate the occupants of a smart-home environment when the occupants are away, such as on vacation. For example, the smart wall switches and/or the smart wall plugs can function as an "occupancy emulator" by learning the occupants' patterns of turning on and off lights, appliances, etc. and mimicking those patterns when the occupants are away.

US2016091217 presents another prior art solution and relates to a system for adjusting environmental conditions based on automatically and manually generated requests. A commissioned unit of US2016091217 comprises at least one IP luminaire which transmits a signal comprising one or more identification codes. An environment control device receives the signal, detects user input indicating one or more preferred environmental conditions, and transmits an environment control request comprising the one or more preferred environmental conditions. An environment manager module receives the environment control request, generates an environment control command using the control request, and transmits the environment control command to one or more commissioned units to alter environmental conditions in a space in accordance with the user input.

EP2919562 presents another prior art lighting solution for controlling a luminaire using control means preprogramed to cause the luminaire to carry out a predefined action as a response to an occurrence of a predefined triggering condition. The solution of EP2919562 comprises storing status indications received in status indication messages from the other devices, analysing the stored status indications in an attempt to identify a sequence of one or more status indications that repeatedly precede an occurrence of said predefined triggering condition, and reprogramming, in response to identifying a sequence of status indications that repeatedly precede an occurrence of said predefined triggering condition, the control means to cause the luminaire to initiate or to carry out a preparatory action for carrying out said predefined action as a response to reception of said identified sequence of status indications.

US2013221858 presents still another prior art solution which relates to an ad-hoc luminaire-controlling system which communicates with nearby luminaires to identify a luminaire cluster. During operation, the system of US2013221858 detects light patterns emitted by one or more remote luminaires and identifies the remote luminaires from the detected light patterns. The system then identifies a luminaire cluster that includes a local luminaire and one or more of the identified remote luminaires. A central or mobile luminaire-controlling system receives a cluster description from one or more luminaires and determines a luminaire topology based on the received cluster descriptions. Each cluster description identifies a set of luminaires that can see each other's light. The central or mobile system can generate a state-modifying command for a set of target luminaires in a cluster based on the luminaire topology and sends the state-modifying command to the target luminaires. The scope of the present invention is defined by the appended claims. All embodiments which do not fall under the scope of the appended claims are examples which are useful to understand the invention, but do not form part of the present invention.

### SUMMARY

It is an object of the present invention to provide a lighting arrangement and lighting system that is able to support the circadian rhythm of people in a space illuminated by the lighting arrangement or lighting system by automatically and autonomously adapting its operation in response to information obtained from its operating environment.

According to the invention, a lighting control device for controlling light output from at least one luminaire is provided, the lighting control device comprising a lighting control logic which defines the manner of controlling light output from the at least one luminaire, and an adaptation means for adjusting the lighting control logic at least in part based on occupancy information received from an occupancy sensor configured to sense motion and/or presence of a person in a certain area. The adaptation means is configured to determine an average switch-on time for a day based on data collected from multiple days by taking into account the collected data about a time the occupancy sensor starts to sense motion on an area of a specific luminaire or a group of luminaires , determine an average duration of a day based on a duration between the time of day the occupancy information starts to indicate presence of a person and the time of day on which occupancy information no longer indicates presence of a person on that day and select between a first lighting mode and a second lighting mode to be used at a certain time interval wherein the selection is based at least in part on the determined average duration of the day and/or the determined average switch-on time, wherein at least one of the following is different in the first lighting mode than in the second lighting mode: the light intensity level produced by the luminaire, color of the light output by the luminaire, color temperature of the light output by the luminaire.

According to the invention, a method for controlling light output from at least one luminaire is provided, the method comprising controlling light output from the at least one luminaire in accordance with a lighting control logic and adjusting the lighting control logic at least in part on occupancy information received from an occupancy sensor which senses motion and/or presence of a person in a certain area. The adjustment comprises determining an average switch-on time for a day based on luminaire data collected from multiple days by taking into account the collected data about a time the occupancy sensor starts to sense motion on an area of a specific luminaire or a group of luminaires , determining an average duration of a day based on a duration between the time of day the occupancy information starts to indicate presence of a person and the time of day on which occupancy information no longer indicates presence of a person on that day and selecting between the first lighting mode and second lighting mode to be used at a certain time interval wherein the selection is based at least in part on the determined average duration of the day and/or the determined average switch-on time, wherein at least one of the following is different in the first lighting mode than in the second lighting mode: the light intensity level produced by the luminaire, color temperature of the light output by the luminaire, color of the light output by the luminaire.

According to the invention, a computer program for lighting control is provided, the computer program comprising computer readable program code configured to cause performing at least the method according to the example embodiment described in the foregoing when said program code is executed on a lighting control device according to the invention.

The computer program according to the above-described example embodiment may be embodied on a volatile or a non-volatile computer-readable record medium, for example as a computer program product comprising at least one computer readable non-transitory medium having the program code stored thereon, which, when executed by one or more computing apparatuses, causes the one or more computing apparatuses at least to perform the method according to the example embodiment described in the foregoing.

In one embodiment of the invention the lighting control device for controlling light output from the luminaire can control multiple luminaires (e.g. in case of a centrally controlled lighting system) in which case it can be arranged to a control unit of the lighting system such as a router of a centrally controlled lighting system. In one embodiment of the invention the lighting control device for controlling light output from the luminaire can control one luminaire (e.g. in case of distributed lighting system) in which case it can be arranged in connection with the luminaire.

With the solution of the invention the lighting system doesn't need information on behavior of a certain person beforehand or configured to the system. Only few parameters are needed for the system when the system is installed and then afterwards the system is able to learn based on sensed motion and time how a specific luminaire should be controlled. Thus, the system is also able to adapt to the changes in the environment and the changes in behavior of the people in the space illuminated by the lighting system.

In the solution of the invention same default parameter values can be used for all luminaires in the installation phase. There's no need for separate configuration of individual luminaires, because individual operation is done by learning. The default parameter values can be set e.g. during the installation phase. The default parameter values can be at least one of the following: light intensity level in the first lighting mode and the second lighting mode (e.g. 80%, 60%), light color in in the first lighting mode and the second lighting mode, light temperature in the first lighting mode and the second lighting mode (e.g. warm/cold temperature) and/or duration how long the change from the first lighting mode to the second lighting mode lasts.

The solution of the invention is well suited e.g. for environments where people are present from the morning to the afternoon or evening such as office buildings and schools where people spend many hours of their days but where configuring of the luminaires according to individual needs of a specific person would be too complicated or it would take too much time.

The exemplifying embodiments of the invention presented in this patent application are not to be interpreted to pose limitations to the applicability of the appended claims. The verb "to comprise" and its derivatives are used in this patent application as an open limitation that does not exclude the existence of also unrecited features. The features described hereinafter are mutually freely combinable unless explicitly stated otherwise.

Some features of the invention are set forth in the appended claims. Aspects of the invention, however, both as to its construction and its method of operation, together with additional objects and advantages thereof, will be best understood from the following description of some example embodiments when read in connection with the accompanying drawings.

### BRIEF DESCRIPTION OF FIGURES

The embodiments of the invention are illustrated by way of example, and not by way of limitation, in the figures of the accompanying drawings, where
Figure 1 presents components and connections of an exemplary embodiment of a centrally controlled lighting arrangement;
Figure 2 presents components of an exemplary embodiment of a distributed lighting arrangement;
Figure 3 illustrates a method according to an example; and
Figures 4A - D illustrate how the operation of the first lighting mode and second lighting mode of a luminaire can be implemented according to different example embodiments.

### DESCRIPTION OF SOME EMBODIMENTS

The lighting arrangement or lighting system of the invention may be arranged for illuminating a space which may comprise e.g. an office room, an open office space, a meeting room, a classroom, an auditorium, a warehouse, a factory or other industrial establishment, etc.

Luminaires which are used in the solution of the invention comprise of at least one light source, a lighting unit controller and a communication unit. The at least one light source of the luminaire can comprise in one embodiment of the invention one or more light emitting diodes (LEDs). The light intensity, color temperature and/or color of the light source of the luminaire can be changed. The lighting unit controller of the luminaire is configured to control the light intensity, color temperature and/or color output of the light source based on the commands or instructions it receives. The solution of the invention also comprises a lighting control device which is configured to receive signals from at least one occupancy sensor and to control or to send commands or instructions (based at least in part on the received signals from the occupancy sensor) to the lighting unit controller controlling the light sources of the luminaire.

The lighting control device, e.g. the lighting control means, comprises lighting control logic which defines the manner of controlling the light output from the luminaire, e.g. switching on and off the light sources of the luminaire and selecting the lighting mode to be used (from the first lighting mode and the second lighting mode) as a response to detecting an occurrence of a triggering condition. The lighting control device comprises also adaptation means which are configured to adjust the lighting control logic. The lighting control device for controlling light output from the luminaire can control one luminaire (e.g. in case of distributed lighting system) in which case it can be arranged within the luminaire or in close connection to the luminaire, for example on top of the luminaire or next to the luminaire. The lighting control device for controlling light output from the luminaire can control multiple luminaires (e.g. in case of a centrally controlled lighting system) in which case it can be a part of a central control unit of the lighting system.

The communication unit of the luminaire may comprise means for a wired connection and/or a wireless connection. It can enable the lighting control device and/or the lighting unit controller to communicate with at least one component of the lighting system, e.g. with other luminaires, occupancy sensors, light sensors, other lighting system units and/or the central controller of the lighting system.

The communication unit of the luminaire or the central controller may comprise a wireless transceiver that is capable of communicating with wireless transceivers using a wireless communication technique or protocol. The wireless communication may be provided by using a suitable short-range wireless communication technique known in the art that enables communication over ranges from a few meters up to a few hundred meters. Examples of suitable wireless communication techniques include Bluetooth, Bluetooth Low-Energy, ZigBee, WLAN/Wi-Fi according to a IEEE 802.11 standard, etc. Further examples include infra-red communications and other non-radio-based short-range communication techniques.

The choice of the short-range wireless communication technique and network topology for a specific embodiment of the lighting system may depend e.g. on the required communication range and/or requirements with respect to energy-efficiency of the communication unit. In one embodiment of the invention, the wireless communication may rely on a wireless mesh network model, for example on a mesh network according to the Bluetooth mesh networking protocol known in the art.

The communication unit of the luminaire or the central controller may comprise a wired transceiver that is capable of communicating with wired transceivers using a wired communication technique or protocol. Wired connections can be implemented for example based on wired network connections or wired bus-type connections. Types of buses which can be used in the solution of the invention can be for example DALI (Digital Addressable Lighting Interface), SDIM (or S-DIM), DMX, RDM (Remote Device Management), KNX, etc.

An occupancy sensor is used in one embodiment of the invention and it is arranged to observe occupancy or non-occupancy in the space and to provide occupancy information, e.g. an occupancy sensor signal, that indicates or otherwise enables deriving the current occupancy status of occupancy or non-occupancy in the space (to be) illuminated by the luminaire. In an example, the occupancy sensor is a motion sensor, such as a passive infrared (PIR) sensor. In this regard, a sensor signal from a motion sensor indicating motion (or lack thereof) may directly serve as or translate into occupancy information, such as an occupancy sensor signal, that indicates occupancy or non-occupancy in the space monitored by the motion sensor. The information from the occupancy sensors of the lighting system can be used in the solution of the present invention to ensure that the lighting schedule corresponds to the circadian rhythms of those affected by the light. The occupancy sensor can be connected using a wired or wireless connection to the lighting control device, e.g. via a communication unit of the luminaire and/or a router.

Also a light sensor can be used in one embodiment of the invention and the light sensor can be arranged to observe ambient light level in the space (to be) illuminated by the luminaire and to provide a light sensor signal that indicates or otherwise enables deriving the current ambient light level in the space illuminated by the luminaire. The light sensor can be connected using a wired or wireless connection to the lighting control device, e.g. via a communication unit of the luminaire and/or a router.

The solution of the invention can be used with different kind of lighting systems. In one embodiment of the invention the lighting system can be a centrally controlled wired and/or wireless lighting system, where the sensor information is processed by a central controller and control commands are received by the luminaire controller. The central controller used in the solution of the present invention can be a router which comprises the lighting control device for controlling light output from the luminaire.

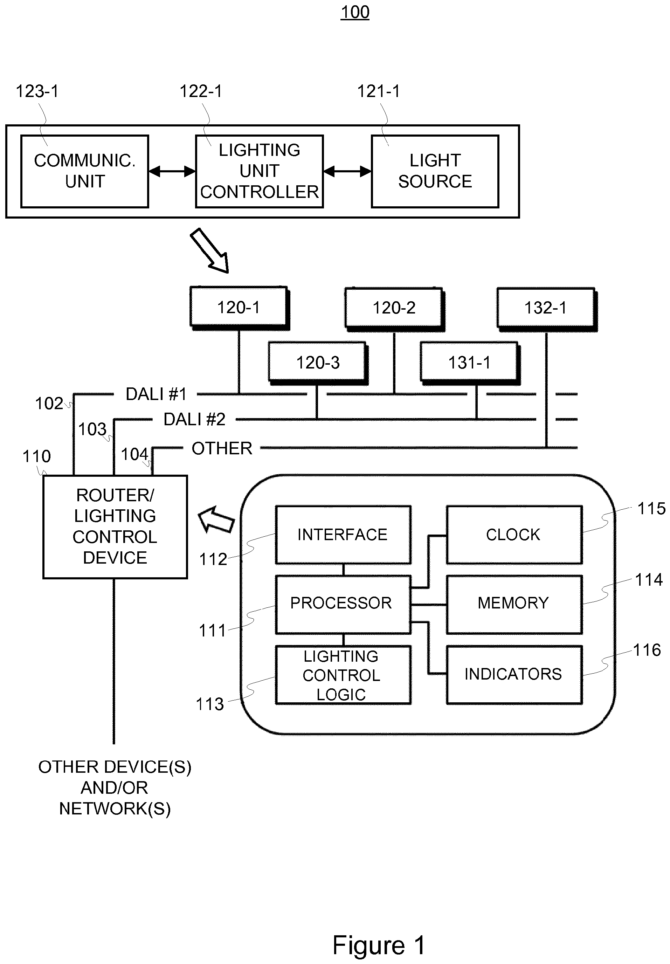

Fig. 1 illustrates one exemplary lighting arrangement which can be used in the solution of the present invention. The lighting arrangement of Fig. 1 is an example of a centrally controlled lighting system.

The lighting arrangement of Figure 1 comprises lighting control device which in this application can be called a router 110 and which is configured to act as a central controller of the lighting system in this example embodiment. The router 110 has connections to one or several buses of the lighting arrangement. Types of buses can be for example DALI (Digital Addressable Lighting Interface), SDIM (or S-DIM), DMX, RDM (Remote Device Management), KNX, etc. In the example of Figure 1, bus 102 is a first DALI-type bus, bus 103 is a second DALI-type bus and bus 104 is other type of bus, e.g. one of the types listed above. Instead of or in addition to wired buses there may be also wireless communication connections to and from the router 110. The router 110 can also have connections to other networks, such as local area networks, or other devices or systems.

Devices of the lighting system, such as luminaires 120-1, 120-2, 120-3, light sensor(s) 131-1 and/or occupancy sensor(s) 132-1 are coupled to the router 110 through the buses and/or the wireless communication connections. The luminaires (an individual luminaire may be referred to via a reference number 120-k) can comprise at least one light source 121-1, a lighting unit controller 122-1 and a communication unit 123-1 as described in the foregoing. In Figure 1, the luminaire 120-1 is used as an example but all the other luminaires of the system (e.g. luminaires 120-2 and 120-3) may have corresponding components. The communication unit 123-1 of the luminaire may comprise a wired connection and/or a wireless connection. The wired or wireless connection may be used for connecting the luminaires 120-k with the router 110 so that instructions can be sent to individual luminaires 120-k from the router 110 and status and/or other information can be received by the router 110 from the luminaires 120-k and/or other components of the lighting system such as light sensor(s) 131-1 and/or occupancy sensor(s) 132-1.

The router 110 can be essentially a special-purpose computer and comprise a processor 111, interface(s) 112 for connection(s) within the lighting system, a memory 114, and a clock 115. The router can also comprise at least one interface for exchanging data related to external control device (not presented in Figure 1 but that interface may be an additional interface or the same as the interface 112). The memory 114 can be used for storing program information, i.e. machine-readable instructions through the execution of which the router 110 manages the lighting system, as well as for storing run-time data. The clock 115 enables the router 110 to link the occupancy information of a certain space to time information and to time the execution of particular instructions with appropriate instances of time, e.g. to select the used lighting mode such as the first lighting mode and the second lighting mode. The clock 115 may also comprise calendar functions. The router 110 may comprise user interface features, such as visual indicators 116. The router also comprises the lighting control logic 113 which defines the manner of controlling the light output from the luminaires, e.g. switching on and off the light sources of the luminaire and selecting the lighting mode to be used, as a response to detecting an occurrence of a triggering condition, as described in the foregoing.

In one embodiment of the invention the lighting system can be a distributed lighting system in which sensor signals go to one or more luminaires located in the vicinity of the sensor and luminaire (controller) independently determines how to react and controls the light accordingly. In one embodiment the luminaires may communicate with other luminaires, but each luminaire controls itself. Luminaires may be able to adjust their behavior based on indications they receive from other luminaires together with the sensor signals they receive. In these kind of distributed systems each luminaire can comprise the lighting control device comprising lighting control logic for controlling light output from the luminaire, and the lighting control device can be arranged in connection with the luminaire.

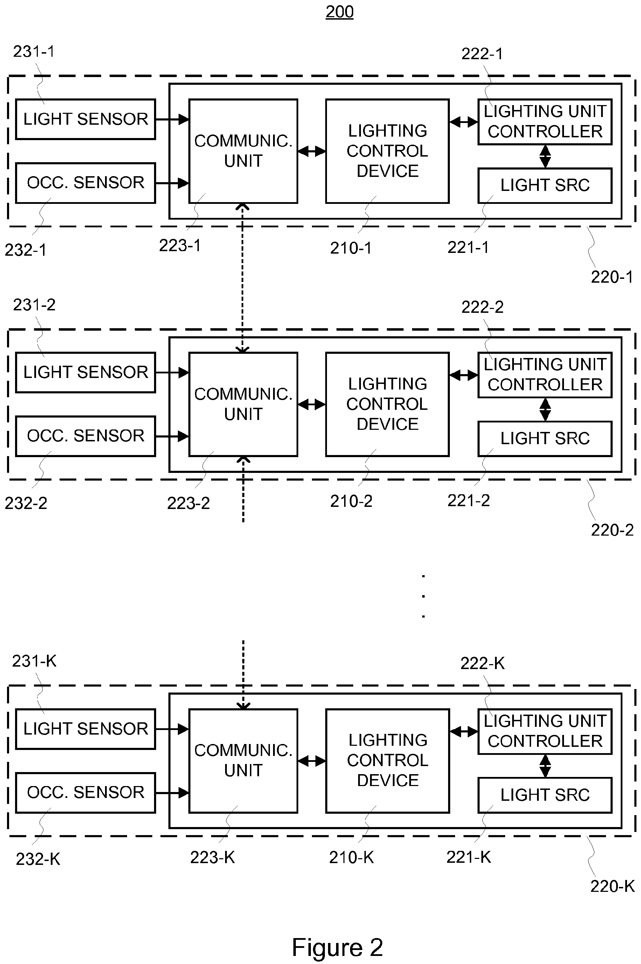

Figure 2 presents an example of a lighting arrangement which comprises luminaires which can be used in a distributed (i.e. not centrally controlled) lighting system.

The lighting arrangement of Figure 2 comprises a plurality of luminaires 220-1, 220-2 ... 220-K. The luminaires of Figure 2 comprise at least one light source 221-1, 221-2 ... 221-K, a lighting unit controller 222-1, 222-2 ... 222-K and a communication unit 223-1, 223-2 ... 223-K as described in the foregoing. Further the luminaires comprise a lighting control device 210-1, 210-2 ... 210-K for controlling the light output from the at least one light source 221-1, 221-2 ... 221-K which lighting control device is configured to control the light sources via the lighting unit controller 222-1, 222-2 ... 222-K. The lighting arrangement also comprises light sensors 231-1, 231-2 ... 231-K and occupancy sensors 232-1, 232-2 ... 232-K, wherein each of the light sensor 231-1, 231-2 ... 231-K and the occupancy sensor 232-1, 232-2 ... 232-K is coupled to the luminaire 220-1, 220-2 ... 220-K, e.g. to communication unit 223-1, 223-2 ... 223-K of the luminaire, via a wired or wireless connection. The communication unit 223-1, 223-2 ... 223-K can also be used for communication with respective control devices of other luminaires 220-1 , 220-2 ... 220-K. The lighting control device 210-1, 210-2 ... 210-K of the luminaire 220-1, 220-2 ... 220-K is configured to send instructions to the lighting unit controller for controlling light output of the luminaire 220-1, 220-2 ... 220-K based at least in part on information received from the light sensor 231-1, 231-2 ... 231-K and/or from the occupancy sensor 232-1, 232-2 ... 232-K. In one embodiment of the invention the lighting control device 210-1, 210-2 ... 210-K is arranged to control light output of the luminaire 220-1 based at least in part on information received from other luminaires 220-2 ... 220-K via the communication unit.

In the example of Figure 2, an individual luminaire may be referred to via a reference number 220-k. In a corresponding manner, an individual light sensor may be referred to via a reference number 231-k, an individual occupancy sensor may be referred to via a reference number 232-k, an individual communication unit of a luminaire may be referred to via a reference number 223-k, an individual lighting control device of a luminaire may be referred to via a reference number 210-k, an individual lighting unit controller of a luminaire may be referred to via a reference number 222-k, and an individual light source of a luminaire may be referred to via a reference number 221-k.

The light sensor 231-k and the occupancy sensor 232-k may be arranged as part of the luminaire 220-k, e.g. arranged in the same housing with the luminaire 220-k, or as a sensor entity that is separate from the luminaire 220-k. In a further example, the light sensor 231-k and the occupancy sensor 232-k may be provided in a sensory assembly that is separate from the luminaire 220-k.

The lighting control logic used in the solution of the invention may be initially defined by configuring the lighting control device e.g. at the manufacturing phase of the lighting control device and/or the luminaire. The lighting control logic may be provided e.g. as program code stored in a memory in the lighting control device that will be executed by a processor in the lighting control device in the course of its operation. In general, the lighting control logic may define one or more pairs of a predefined triggering condition and a lighting control action to be carried out as a response to an occurrence of the predefined triggering condition, where the triggering condition may be based, for example (at least one of the following), on the light sensor signal, on occupancy information, e.g. an occupancy sensor signal, on an external sensor signal, on a status indication received e.g. from lighting control device of another luminaire, on a command from the lighting control device, defined average switch on time, defined average duration of the day.

The lighting control device may be arranged to control the light output of the luminaire or a plurality of luminaires with the lighting control logic based the at least in part on the light sensor signal and/or the occupancy information such as occupancy sensor signal. Examples of such pairs of a triggering condition and a lighting control action to be taken as a response to an occurrence of the triggering condition may include the following:
- The lighting control device can be arranged to, as a response to the occupancy information indicating occupancy after a period of non-occupancy, switch on the light output from the luminaire(s) at a target light intensity. The target light intensity can be e.g. the light intensity level of the first lighting mode and/or the second lighting mode based on the lighting mode used at that time interval. This can be considered an active state of the luminaire.
- The lighting control device may be arranged to, as a response to the occupancy information indicating continuous occupancy, keep the light output from the luminaire(s) at the current light intensity, e.g. at the target light intensity. The target light intensity can be e.g. the light intensity level of the first lighting mode and/or the second lighting mode based on the lighting mode used at that time interval. Also in this case the luminaire can be considered to be in the active state.
- The lighting control device may be arranged to, as a response to the occupancy information indicating non-occupancy after a period of occupancy, start a first switch-off timer to measure a switch-off period, to start a dim-down timer to measure a dim-down period, to adjust the light output from the luminaire(s) to an intermediate light intensity in response to the dim-down timer elapsing before the occupancy information again indicating occupancy and to adjust the light output from the luminaire to a stand-by light intensity in response to the switch-off timer elapsing before the occupancy information indicating occupancy. The state when no occupancy is determined and/or stand-by light intensity is used can be considered a passive state.

In one embodiment of the invention, especially in a case of a distributed lighting system, the lighting control device can be arranged to transmit status indication messages in the course of its operation via the communication unit. Each status indication message can comprise, in addition to information defined by the applied communication protocol, one or more status indications relating to the current or recent operating status of the lighting control device transmitting the status indication message. The lighting control device receives status indication messages (and hence status indications) from other luminaires and/or lighting control devices of the lighting system. A status indication transmitted from the luminaire and/or the lighting control device or received at the luminaire and/or the lighting control device may comprise an action indication that identifies a lighting control action taken by the lighting control device that has transmitted the status indication, e.g. using the first lighting mode and/or using the second lighting mode by the luminaire. Status indication messages can be sent e.g. in the case of a distributed lighting system to other luminaires. The status indications can be sent e.g. via the communication techniques described in the foregoing.

In the solution of the invention the system and/or individual luminaires require time information which can be sent e.g. via a router, a control unit, or a device such as a mobile phone or computer. The time information can comprise time and/or calendar information, such as current time.

As already stated with in the foregoing, the lighting control device of the present invention comprises lighting control logic. The solution of the invention also comprises an adaptation means for adjusting the lighting control logic at least in part based on occupancy information received from an occupancy sensor configured to sense motion and/or presence of a person in a certain area. In the solution of the invention the lighting control device is configured to determine an average switch-on time for a day based at least in part on the time of day the occupancy information (from e.g. occupancy sensor) starts to indicate motion and/or presence of a person. The lighting control device is also configured to determine an average duration of the day based at least in part on e.g. how long the occupancy information (e.g. from occupancy sensor) indicates motion and/or presence of a person during the day. The average duration of the day may be determined e.g. based on the duration between the time of day the occupancy information starts to indicate motion and/or presence of a person and the time of day on which motion and/or presence of a person is no more indicated by the occupancy information. This way the system is able to collect information relating to the circadian rhythm of the people in the space.

The lighting control device is further configured to select between a first lighting mode and a second lighting mode to be used at a certain time interval based at least in part on the determined average duration of the day and/or the determined average switch-on time, wherein at least one of the following is different in the first lighting mode than in the second lighting mode: the light intensity level produced by the luminaire, color of the light output by the luminaire, color temperature of the light output by the luminaire. In one embodiment of the invention the lighting control device is configured to activate the first lighting mode at or before the determined average switch on time and/or change from the first lighting mode to the second lighting mode after a predefined duration from the determined average switch-on time. In one embodiment of the invention the predefined duration can be based at least in part on the determined average duration of the day. In one embodiment of the invention the predefined duration can be shorter than or equal to the determined average duration of the day. This way the system is able to configure the light output of the luminaires to support the detected circadian rhythm by providing different kind of light at different time of the day.

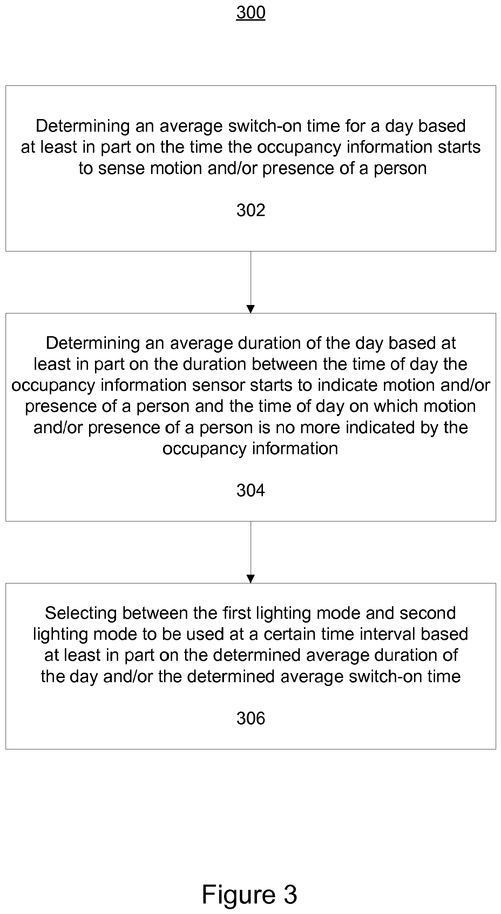

The operations described in the foregoing may be also defined as a steps of method. As an example in this regard, Figure 3 depicts a flowchart that illustrates a method 300 for controlling light output from the luminaire. The operations described with references to blocks 302 to 306 pertaining to the method 300 may be varied or complemented in a number of ways, for example as described in the foregoing or in the following in context of the lighting control device and/or the luminaire.

In the method 300 light output from at least one luminaire is controlled in accordance with a lighting control logic and the lighting control logic is adjusted at least in part on the information received from an occupancy sensor which senses motion and/or presence of a person in a certain area. The adjustment comprises the step 302 of determining an average switch-on time for a day based at least in part on the time the occupancy information starts to indicate motion and/or presence of a person and step 304 of determining an average duration of the day based at least in part on the duration between the time of day the occupancy information starts to indicate motion and/or presence of a person and the time of day on which motion and/or presence of a person is no more indicated by the occupancy information. Next the method can proceed to step 306 of selecting between the first lighting mode and second lighting mode to be used at a certain time interval based at least in part on the determined average duration of the day and/or the determined average switch-on time, wherein at least one of the following is different in the first lighting mode than in the second lighting mode: the light intensity level produced by the luminaire, color temperature of the light output by the luminaire, color of the light output by the luminaire.

In the solution of the invention the lighting system designer may design light intensity levels, color of the light output by the luminaire and/or color temperature of the light output by the luminaire. The different light intensity levels, color of the light output by the luminaire and/or color temperature of the light output by the luminaire can be stored to the system and/or the luminaire at least for the first lighting mode and the second lighting mode.

In one embodiment of the invention a lighting system (e.g. a centrally controlled lighting system) may have a pre-installed profile for adjusting the light as a function of time. The lighting system can adjust the start- and stop -times of the pre-programmed lighting profile of the lighting system based at least in part on information from the occupancy sensor in accordance to lighting control logic. This way the lighting system installer does not need to know details of the environment, e.g. whether the working day in the premises typically starts at 6 am or 10 am. The information of the occupancy sensor can be used to create a profile for a lighting system (e.g. a centrally controlled lighting system) based on the typical usage hours of the premises.

In one embodiment of the invention a lighting system (e.g. a distributed lighting system) may have different default values for the light (to be) produced by the luminaire at different times of the day. The information from the occupancy sensor can in this case be used in determining which are the default lighting settings to be used in the lighting system for a specific luminaire.

In one embodiment of the invention the light intensity of the luminaire is different in the first lighting mode and in the second lighting mode, e.g. so that light intensity is higher in the first lighting mode than in the second lighting mode. The light intensity can be selected this way because the need for light is higher in the morning and lower in the evening and this kind of lighting can thus be used to support maintaining the circadian rhythm. In one embodiment of the invention light intensity is higher in the second lighting mode than in the first lighting mode. This embodiment can be used e.g. for producing wake up light in the morning and then changing to the higher intensity level after a certain duration.

The target light intensity may be defined as a predefined percentage of the maximum light intensity of the at least one light source. In one example embodiment the first lighting mode can have light intensity value of 80 - 90% and the second lighting mode light intensity value of 60% - 70%.

In one embodiment of the invention the first lighting mode and the second lighting mode comprise different color and/or color temperature produced by the luminaire, e.g. so that the color temperature produced by the luminaire is higher, i.e. colder, in the first lighting mode than in the second lighting mode. Also this kind of selection of the light produced by the luminaires can be used to support the circadian rhythm because e.g. wavelengths of the blue light may affect the sleep. In one embodiment of the invention the color temperature produced by the luminaire is lower, i.e. warmer, in the first lighting mode than in the second lighting mode. This embodiment can be used e.g. for producing wake up light in the morning and then changing to colder color temperature after a certain duration.

In one embodiment of the invention the lighting modes can also comprise a combination of the above embodiments for example different intensity levels and different color temperatures in the first lighting mode and the second lighting mode.

In one embodiment of the invention there may be further lighting modes in addition to the first lighting mode and the second lighting mode. All the lighting modes have at least one of the following lighting characteristics different between each other: the light intensity level produced by the luminaire, color of the light output by the luminaire, color temperature of the light output by the luminaire.

In one embodiment of the invention the solution of the invention comprises a third lighting mode. In one embodiment of the invention the third lighting mode can be used for example as a lighting mode following the second lighting mode. The third lighting mode can be used e.g. in the evening.

In the embodiment with the first, second and third lighting mode, the lighting control device is configured, in addition to using the first lighting mode and the second lighting mode at a certain time interval, to select also when the third lighting mode is used based at least in part on the determined average duration of the day and/or the determined average switch-on time. In one embodiment of the invention the first lighting mode is used in the beginning of the day when occupancy is detected, the second lighting mode is used after the first lighting mode and the third lighting mode is used the after the second lighting mode in the end of the day as long as occupancy is sensed. The change from the second lighting mode to the third lighting mode can be implemented in a similar manner as the change from the first lighting mode to the second lighting mode as presented in the examples of this patent application.

In one embodiment of the invention there's no need for different operation modes for certain weekdays. In this case the lighting system can use the same determined average switch on time and average duration of the day every day for a specific luminaire or group of luminaires. This kind of solution can be used to help to keep the day rhythm similar every day. For the same reason same settings can be used also on weekends so that also then the lighting system can help to keep the normal rhythm.

The luminaires used in the solution of the invention can be switched on to active state, e.g. the target light level of the luminaire, when motion is sensed, as described in the foregoing. The selected mode, e.g. the first lighting mode or the second lighting mode, used at that time specifies the color, color temperature and/or light intensity level. During breaks (when no occupancy is detected) the luminaire or luminaires can be switched to stand-by or passive state but as soon as motion is detected and motion and/or presence in the space continues, same mode of operation can be resumed, i.e. the same lighting mode can be used as before the passive state and/or the lighting mode can be changed to the second lighting state from the first lighting state if this change is determined to be carried out during the passive state. This way the energy consumption of the luminaires can be minimized.

In one embodiment of the invention the lighting control device may be further arranged to switch on the light output from the luminaire in response to the luminaire receiving operating power (e.g. from the mains electricity) after a period of not receiving the operating power. In this case, in one embodiment of the invention, the lighting control device may select the used lighting mode based on a predefined setting before it receives correct time information, e.g. the first lighting mode can be selected after a power outage before receiving correct or actual time information. As soon as the correct or actual time information is received, the lighting control device can select the correct lighting mode used at that specific time.

In one embodiment of the invention the lighting control device is configured to redefine the average switch-on time and/or the average duration of the day based on the information received from the occupancy sensor if there is deviation between the determined average switch-on time and/or the determined average duration of the day and information received from the occupancy sensor. Based on this the lighting system is able to adapt its operation in relation to the space and to the circadian rhythm of the people in the space.

The adaptation means in the lighting control device may be arranged to adjust or update the (preprogrammed) lighting control logic defined for the lighting control device based at least in part on the determined average switch on time and average duration of the day and/or light intensity level, color temperature of the light and/or color in the first lighting mode and in the second lighting mode. The adjusting or updating of the preprogrammed lighting control logic defined for the lighting control device can be done e.g. when at least one of the determined values, such as the determined average switch on time and average duration of the day and/or light intensity level, color temperature of the light and/or color in the first lighting mode and in the second lighting mode, are updated. Thus the operation of the luminaire can be adapted to the environment and changes in the environment. This way the lighting system is learning the usual circadian rhythm of the people and is also able to adapt its operation if the there are changes in the behavior of the people in the space, e.g. when the circadian rhythm of the people in the space changes.

The lighting control device may be arranged to store in a memory a history of obtained sensor data and/or data determined from the sensor data, e.g. average switch on time, average duration of the day, occupancy indications, such as occupancy period or non-occupancy period, starting times and end-times of these periods, derived based on the occupancy information. In this regard, the observed indications and changes may be stored at predefined time intervals and/or each time a change in occupancy status (from non-occupancy to occupancy or from occupancy to non-occupancy) is observed.

In one embodiment of the invention average switch on time and average duration of the day can be determined by an analysis for identification of a repeatedly occurring pattern. If e.g. the occupancy sensor starts to sense motion and presence at certain time of the day, this can be used to define the average switch on time. During the operation of the lighting system and/or the luminaire data can be collected from multiple days and the average switch on time can be determined by taking into account the collected data about the time the occupancy sensor starts to sense motion on the area of a specific luminaire or a group of luminaires. If e.g. the motion and/or presence sensed by the occupancy sensor stops at certain time, this time can be used to determine the average duration of the day (e.g. with information regarding the average switch on time also available). During operation of the lighting system and/or the luminaire data can be collected from multiple days and the average duration of the day can be determined by taking into account the collected data about the time the presence and or motion sensed occupancy sensor stops on the area of a specific luminaire or a group of luminaires.

In one embodiment of the invention the lighting control device can collect the information from e.g. occupancy sensor(s) and carry out the determinations of e.g. average switch on time, average duration of the day and time when the first lighting mode is to be used, time when the second lighting mode is to be used and/or the time when the change from the first lighting mode to the second lighting mode is carried out or started. The lighting control device can send at least some of this collected and/or determined information to an external device, an external server and/or to an external service such as a cloud service. In one embodiment of the invention the lighting control device can collect the information and the determination of e.g. at least one of the values mentioned above can be carried out in external device, an external server and/or an external service such as a cloud service which can then send the determined values to the lighting control device.

In the solution of the invention different luminaires can be in different modes at the same time based on the sensed motion and/or presence of a person on the area of the specific luminaires. The different luminaires can have different determined average duration of the day and different average switch on time based on sensed presence or motion on the area of a specific luminaire. This way the lighting system can support to the circadian rhythm of the persons usually present in the area of a specific luminaire.

In one example case, a meeting room can be usually in use at 10.00 and this way the determined average switch on time can be set for 10.00 for the luminaires in this space. For example, a flexi office space (at least certain part of it) can be in use e.g. from 8.00 and this way the determined average switch on time can be set for 8.00 for the luminaires in this space (or the part of the space where people tend to come first). In the same example concerning the meeting room, the meeting room can be in use usually until 16.00 and thereby the average duration of the day can be determined to be 6 hours. In the same example concerning the flexi office space, the space can be in use usually until 17.00, thereby the average duration of the day can be determined to be 9 hours (for at least certain part of the space). In flexi office space the different luminaires can have different determined average duration of the day and different average switch on time based on sensed presence or motion on the area of a specific luminaire.

In one embodiment of the invention if certain detected motion if not regular, (i.e. detected number of events is under certain number of times, e.g. 1 triggering event) it doesn't have to be used in determining average switch on time or average duration of the day.

In one embodiment of the invention a short motion that does not continue long is not used when determining average switch on time or average duration of the day. This can be implemented e.g. so that if detected motion has a duration shorter than a certain time limit, the sensed motion is not taken into account when determining average switch on time or average duration of the day. These kinds of circumstances can happen e.g. if a cleaner is visiting the space or a person is just walking by. Also in this case the luminaires are preferably switched to the active state even if this observed occupancy is not taken into account when defining average on time and/or average duration of the day.

In one embodiment of the invention the occupation sensor is able, instead of only informing whether someone is in the area, to determine the occupation level of the area. In one embodiment of the invention the occupancy can be defined in certain number of levels, e.g. in three different levels (in which case the levels can be e.g. there are only a few people, there are medium number of people, and there are many people in the area). The occupancy level information can be used as an input for the lighting system, e.g. if there are only few people in the area, the change from first mode to the second mode can be done earlier than in the normal case and if there are lot of people in the area, the change from first mode to the second mode can be done later as in the normal case. Examples of these kinds of occupation sensors which can be utilized to sense the occupation level are cameras or radars. This kind of occupancy level information can be utilized also for the energy saving purposes. One example of this kind of circumstances is a case in which only few people are at the office before certain time (e.g. 8 am) and more people arrive after that time. The light output of the luminaires can be set in the reduced level (e.g. 70% level) during the period before the occupation level reaches a predefined level (e.g. before 8 am), and e.g. to higher level (e.g. 80-90% and/or full level) when the occupation level reaches a predefined level (e.g. after 8 am).

In one embodiment of the invention other luminaires being close to a specific luminaire, e.g. within a predefined distance and/or in same space, can affect how the lighting modes of a specific luminaire are used. If there are other luminaires close to the specific luminaire in different mode e.g. the duration of the mode change from the first lighting mode to the second lighting mode can be changed for the specific luminaire, for example decreased, and/or the light intensity, color temperature and/or color can be matched towards the other luminaire being close to the specific luminaire. The specific luminaire can receive information on the status of the other luminaires based on the status indication messages sent by the other luminaire, e.g. in a distributed system. In the case of a centrally controlled system the lighting control device, such as the router, has the information of the used lighting mode of a specific luminaire and it can take into account the lighting modes used at a specific time by luminaires that are close to each other. In one example embodiment light intensity can be adjusted so that if usually levels 90% and 60% for the luminaires in different modes (one being in the first lighting mode and the second being in the second lighting mode) would be selected, in this case with luminaires being close to each other but in different modes, levels 80% and 70% can be used.

The lighting control device may be further arranged to control the light output from the luminaire in accordance with one or more control signals received from a user-operable control panel arranged in the space (to be) illuminated by the luminaire. The control panel may be coupled to the lighting control device via wired or wireless communication. In this regard, the control panel may enable user actions for switching the lights on and setting the lights off and possibly also for adjusting the light produced by the luminaire to a desired light intensity, desired color and/or desired color temperature. The lighting control device may be arranged to control the light output based on the one or more control signals from the control panel, e.g. to switch on the light output from the luminaire (at the target light intensity of the first lighting mode and the second lighting mode), to adjust the light output from the luminaire to the stand-by light intensity (e.g. switch off the light output) and/or to adjust the light output from the luminaire to a user-selected light intensity.

The lighting control device may be further arranged to switch on the light output from the luminaire in response to a command or message received from a remote control device, which may comprise e.g. a dedicated remote control device or a mobile communication device (such as a mobile phone or a tablet computer) provided with a software application that provides the remote control functionality. The remote control device may enable similar control of the light output from the luminaire as described above for the control panel, whereas the lighting control device may receive the one or more control signals from the remote control device via wireless communication.

The selection by the user can be used in some embodiments as an input for the learning, e.g. in determination of the average switch on time, average duration of the day and/or time when the first lighting mode is changed to the second lighting mode. There can be some system parameters for the learning, e.g. how many times the user has to activate a certain event so that the system is learning from it, for example how many user input repetitions (e.g. on how many different days) are required for redefining when the first lighting mode is used and when the second lighting mode is used.

A user controllable control panel or device, such as button, can be used e.g. to increase or decrease the light level for a certain duration, e.g. 15 minutes. In the long term, using the button or non-using the button can be used to affect the duration how long the first lighting mode is used before changing to the second lighting mode. In one example case, if the button for increasing/decreasing the light level is pushed in a repetitive manner on different days, e.g. one or more times on different days, when the lighting system changes to the second lighting mode, the duration how long the first lighting mode is used, can be changed (i.e. the change from the first lighting mode to the second lighting mode can be done earlier or later as it would be normally be done).

Having user operable control panel or device can be useful for example in a classroom or in a meeting room if a video or presentation is watched so that the light levels can be dimmed for the duration of the video or the presentation. In one embodiment of the invention this selection, e.g. dimming or increasing light intensity level, can be used e.g. for a duration of certain time or until next break, sensed by the occupancy sensor.

In one embodiment of the invention the change from the first lighting mode to the second lighting mode is configured to last a certain duration, during which the light intensity, color and/or color temperature of the luminaire is changed from the levels of the first lighting mode towards the second lighting mode continuously, gradually and/or in steps. In this case during the change the light intensity, color and/or color temperature levels can be between the levels of the first lighting mode and the second lighting mode.

In one embodiment of the invention the lighting control device is configured to keep the light intensity, color and/or color temperature of the luminaire essentially constant on the active state of the luminaire and to change the light intensity, color and/or color temperature from the levels of the first lighting mode towards the second lighting mode only after the luminaire has been in passive state and enters again active state. In this case during the change the light intensity, color and/or color temperature levels can be between the levels of the first lighting mode and the second lighting mode. In one embodiment of the invention the change from the first lighting mode and the second lighting mode can be done directly from the first lighting mode to the second lighting mode so that after a passive state of the luminaire the second lighting mode is used. In one embodiment of the invention the change from the first lighting mode and the second lighting mode can be done in steps during the passive state of the luminaire, e.g. so that after the passive state a light intensity level, color and/or color temperature is used which is between the first lighting mode and the second lighting mode and after the next passive state a light intensity level, color and/or color temperature is again changed towards the state of the second lighting mode but it can be still between the levels of the first lighting mode and the second lighting mode.

In one embodiment of the invention change from the first lighting mode to the second lighting mode is determined based at least in part on the average duration of the day and/or durations and/or frequency of the passive states between the active states of the luminaire.

In one embodiment of the invention the lighting control device is configured to start the change from the first lighting mode to the second lighting mode already at the determined average switch-on time. In one embodiment of the invention the change from the first lighting mode to the second lighting mode can have essentially similar duration as the determined average duration of the day. In this case the light of the luminaire can changes e.g. during the determined day from the levels of the first lighting mode to the levels of the second lighting mode.

In one embodiment of the lighting system of the invention there can be predefined settings how a luminaire adapts to different kind of spaces and environments. These setting may e.g. define how and/or when the change from one lighting mode to another lighting mode is carried out. The settings to be used can be selected e.g. based on the detected and determined average switch on time, average duration of the day and or durations and/or frequency of the passive states between the active states of the luminaire.

In the following paragraph some example embodiments are described relating to different environments and different conditions and how the lighting control logic can adapt for these kind of environments.

In one embodiment of the invention for a space with a long determined duration of the day and without interruptions for a long time (e.g. many hours), the first lighting mode can be used for longer than e.g. 5 hours or 8 hours after which a slow dimming or change of the lighting mode from first mode to the second mode can be done, e.g. when no occupancy is detected or when luminaires are switched on again after a passive (non-occupancy) period.

In one embodiment of the invention for a space with a short determined duration of the day and long periods of non-occupancy between active states, the second lighting mode can be used essentially always, e.g. light intensity level set to lower intensity level, e.g. 60%-level. "Short" can be in one embodiment of the invention defined as 1 - 10 min. In this scenario the active state of the luminaire can be shorter than the passive (non-occupation) state. These kind of spaces can be e.g. cleaning closets, copy rooms, etc.

In one embodiment of the invention for a space with a short determined duration of the day and short periods of non-occupancy between active states, change from the first lighting mode to the second lighting mode can be done during e.g. 8 hours from the average switch on time for example so that light level of every active state stays constant and the level is changed towards the levels of the second lighting mode when the luminaires are activated after a non-occupancy period, i.e. during or after the passive periods. These kind of spaces can be e.g. hallways, stairs.

In one embodiment of the invention for a space with a medium determined duration of the day and short periods of non-occupancy between active states the change from the first lighting mode to second lighting mode can be configured in the similar way as in the previous example, i.e. the change from the first lighting mode to the second lighting mode during certain time, e.g. 8 hours, from the average switch on time so that e.g. light level of every active state stays constant and the level is decreased after the luminaires are activated after a passive (non-occupancy) period. These kind of spaces can be e.g. classrooms and some meeting rooms. In this case a short break can cause e.g. the intensity level to be decreased, e.g. from 83%->76%. Another possibility is to change the intensity level in steps, e.g. during the first active state a certain level, for example 80%, is used and during the second active state a certain (e.g. lower) level, for example 70% is used.

In one embodiment of the invention for a space with a medium determined duration of the day and long periods of non-occupancy between active states the first lighting mode can be used for certain duration, e.g. 5 hours, and then the lighting mode can be changed to the second lighting mode. These kind of spaces can be for example some meeting rooms.

In one embodiment of the invention seasons can have effect on the used light intensity level of a specific lighting mode, the duration of the change of the lighting mode and/or the time when lighting mode is changed from first mode to the second mode. The need for light of a person is higher in the winter and therefore the lighting system can be configured to shorten the duration of the first mode and/or advance the change from the first mode to the second mode in summer and spring when compared to the winter and fall. In this case the lighting designer can set the parameters of the system and the plan e.g. according to summertime values and the lighting system can adjust the values, e.g. increase the light level for the winter. In one embodiment the lighting system can also be configured the other way around, i.e. the parameters of the lighting system and the plan can be set e.g. according to wintertime values and the lighting system can adjust the values, e.g. decrease the light level for the summer.

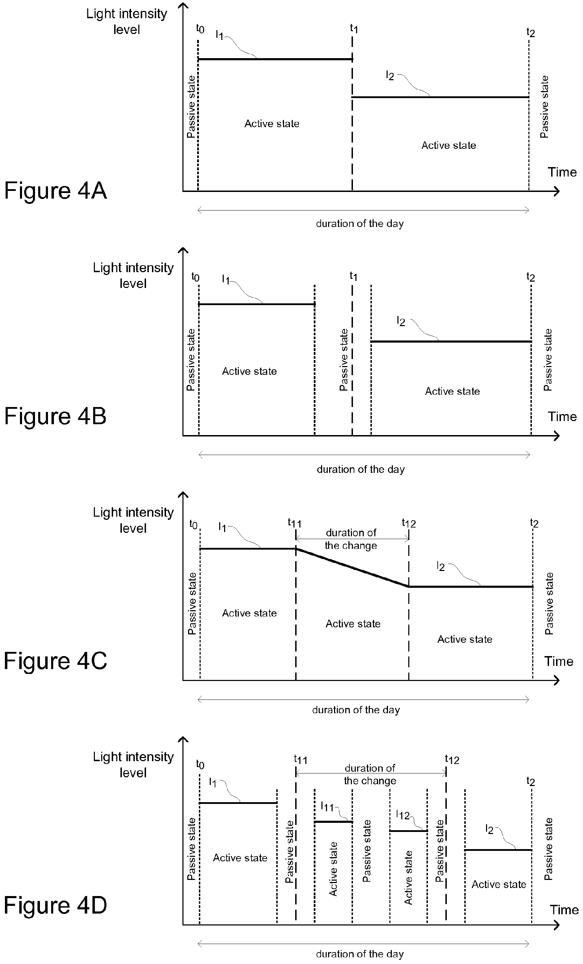

Figures 4A - D present example implementations of different embodiments of the solution of the invention. These figures illustrate how the different lighting modes can be used and how the change from the first mode to the second mode can be implemented. In the figures 4A - D the light intensity level of a specific luminaire is presented on one axis in relation to time on the other axis.

Figure 4A presents a situation when there is occupancy in the area of the luminaire during the whole day between t₀ and t₂. Thus, the luminaire is in the active state for the whole duration of the day. Between time t₀ and t₁ the luminaire is in the first lighting state, which in this example means that the luminaire produces light intensity l₁ as the light intensity of the first lighting mode. Between time t₁ and t₂ the luminaire is in the second lighting state, which in this example means that the luminaire produces light intensity l₂ as the light intensity of the second lighting mode. In this example scenario the luminaire changes the light intensity from the light intensity level of the first lighting mode l₁ to the light intensity level of the second lighting mode l₂ at the time t₁.

Figure 4B presents a situation when there is occupancy and non-occupancy in the area of the luminaire during the day. Thus, the luminaire is in the active state always when occupancy is detected. Between time t₀ and t₁ the luminaire is in the first lighting state, which in this example means that the luminaire produces light intensity l₁ as the light intensity of the first lighting mode when occupancy is detected. Between time t₁ and t₂ the luminaire is in the second lighting state, which in this example means that the luminaire produces light intensity l₂ as the light intensity of the second lighting mode when occupancy is detected. In this example scenario the luminaire changes the light intensity to the light intensity level of the second lighting mode l₂ after t₁ when the passive state of the luminaire ends, and occupancy is sensed once again.

Figure 4C presents a situation when there is occupancy in the area of the luminaire during the whole day between t₀ and t₂. Thus, the luminaire is in the active state for the whole duration of the day. Between time t₀ and t₁₁ the luminaire is in the first lighting state, which in this example means that the luminaire produces light intensity l₁ as the light intensity of the first lighting mode. Between time t₁₂ and t₂ the luminaire is in the second lighting state, which in this example means that the luminaire produces light intensity l₂ as the light intensity of the second lighting mode. In this example scenario the luminaire changes the light intensity in a continuous manner from the light intensity level of the first lighting mode l₁ to the light intensity level of the second lighting mode l₂ during t₁₁ and t₁₂.

Figure 4D presents a situation when there is occupancy and non-occupancy in the area of the luminaire during the day. Thus, the luminaire is in the active state always when occupancy is detected. Between time t₀ and t₁₁ the luminaire is in the first lighting state, which in this example means that the luminaire produces light intensity l₁ as the light intensity of the first lighting mode when occupancy is detected. Between time t₁₂ and t₂ the luminaire is in the second lighting state, which in this example means that the luminaire produces light intensity l₂ as the light intensity of the second lighting mode when occupancy is detected. In this example scenario the luminaire starts the mode change from the first lighting mode to the second lighting mode at time t₁₁. In this example case the light intensity levels are changed in multiple steps towards the light intensity level of the second lighting mode l₂ so that during the change the light intensity level is kept constant during an active state of the luminaire and after a passive state of the luminaire after t₁₁ the light intensity level is changed towards the light intensity level of the second lighting mode to light intensity level l₁₁ . After the next passive state of the luminaire the light intensity level is changed further towards the light intensity level of the second lighting mode to light intensity level l₁₂. After the next passive state after t₁₂ the light intensity level is changed to the level l₂ of the second lighting state when occupancy is detected.

Reference(s) to a processor in this application should not be understood to encompass only programmable processors, but also dedicated circuits such as field-programmable gate arrays (FPGA), application specific circuits (ASIC), signal processors, etc. Features described in the preceding description may be used in combinations other than the combinations explicitly described.

In the present disclosure, although functions have been described with reference to certain features, those functions may be performable by other features whether described or not. Although features have been described with reference to certain embodiments, those features may also be present in other embodiments whether described or not.

## Claims

1. A lighting control device for controlling light output from at least one luminaire (120-k, 220-k), the lighting control device (110, 210-k) comprising:
a lighting control logic for defining the manner of controlling light output from the at least one luminaire (120-k, 220-k), the lighting control device **characterized by**:
an adaptation means for adjusting the lighting control logic at least in part based on occupancy information received from an occupancy sensor (132, 232-k) configured to sense motion and/or presence of a person in a certain area, wherein the adaptation means is configured to:
- receive time information,
- determine an average switch-on time for a day based on luminaire data collected from multiple days by taking into account the collected data about a time the occupancy sensor starts to sense motion on an area of a specific luminaire or a group of luminaires, and
- determine an average duration of a day based on a duration between the time of day the occupancy information starts to indicate presence of a person and the time of day on which the occupancy information no longer indicates presence of a person on that day and
wherein the lighting control device is configured to select between a first lighting mode and a second lighting mode to be used at a certain time interval wherein the selection is based at least in part on the determined average duration of the day and/or the determined average switch-on time, wherein at least one of the following is different in the first lighting mode than in the second lighting mode: the light intensity level produced by the luminaire (120-k, 220-k), color of the light output by the luminaire (120-k, 220-k), color temperature of the light output by the luminaire (120-k, 220-k).

2. A lighting control device according to claim 1, wherein the lighting control device (110, 210-k) is configured to activate the first lighting mode at or before the determined average switch on time and/or to change from the first lighting mode to the second lighting mode after a predefined duration from the determined average switch-on time, wherein the predefined duration is based at least in part on the determined average duration of the day, and wherein the predefined duration is shorter than or equal to the determined average duration of the day.

3. A lighting control device according to claim 1 or 2, wherein the lighting control device (110, 210-k) is configured to redefine the average switch-on time and/or the average duration of the day based on the occupancy information if there is deviation between the determined average switch-on time and/or the determined average duration of the day and occupancy information.

4. A lighting control device according to any preceding claim, wherein the light intensity produced by the luminaire (120-k, 220-k) is higher in the first lighting mode than in the second lighting mode, and/or the color temperature produced by the luminaire (120-k, 220-k) is colder in the first lighting mode than in the second lighting mode.

5. A lighting control device according to any preceding claim, wherein the lighting control device comprises a third lighting mode and/or the lighting control device is configured to select when the third lighting mode is used based at least in part on the determined average duration of the day and/or the determined average switch-on time.

6. A lighting control device according to any preceding claim, wherein the light intensity, color and/or color temperature produced by the luminaire (120-k, 220-k) in the first lighting mode and in the second lighting mode are predefined and/or stored into the memory (114, 415) of the lighting control device (110, 210-k).

7. A lighting control device according to any preceding claim, wherein the lighting control device (110, 210-k) is configured to receive time information from a gateway unit, a control unit and/or an external device such as a mobile phone or a computer.

8. A lighting control device according to any preceding claim, wherein the lighting control device (110, 210-k) is configured to adjust the light output from the luminaire (220-k) based on the occupancy information so that light sources (121-1, 221-k) are switched to passive state after a continuous period of a predetermined duration during which occupancy information does not indicate persons present in the monitored area, and
the light sources (121-1, 221-k) are switched to an active state after occupancy of a person is indicated after non-occupancy period, wherein in the active state the lighting control device is configured to select the lighting mode of the luminaire based on which mode is the current lighting mode.

9. A lighting control device according to any preceding claim, wherein the change from the first lighting mode to the second lighting mode is configured to last a certain duration, during which the light intensity, color and/or color temperature of the luminaire (120-k, 220-k) is changed from the levels of the first lighting mode towards the second lighting mode continuously, gradually and/or in steps.

10. A lighting control device according to claim 9 wherein the light intensity, color and/or color temperature of the luminaire (120-k, 220-k) is kept essentially constant on the active state of the luminaire and the light intensity, color and/or color temperature is configured to be changed from the levels of the first lighting mode towards the second lighting mode only after the luminaire has been in passive state and enters again active state.

11. A lighting control device according to claims 9 or 10, wherein the duration of the change from the first lighting mode to the second lighting mode is configured to be determined based at least in part on the average duration of the day and/or durations and/or frequency of the passive states between the active states of the luminaire (120-k, 220-k).

12. A lighting control device according to any preceding claim, wherein the luminaire (120-k, 220-k) comprises a communication unit (123-1, 223-k) for communication with a lighting control device (110) of the lighting system and/or lighting control devices (210-k) that serve to control other luminaires (120-k, 220-k), the communication unit arranged to receive status indications from the at least one lighting control device (110, 210-k), the status indications comprising at least the lighting mode used by the luminaire (120-k, 220-k).

13. A lighting control device according to any preceding claim, wherein the lighting control device (110, 210-k) is configured to match the light intensity levels, color and/or color temperature towards the light intensity levels, color and/or color temperature of the other luminaires (120-k, 220-k) that are next to the luminaire (120-k, 220-k) and/or within a predetermined distance from the luminaire (120-k, 220-k).

14. A lighting control device according to any preceding claim, wherein the lighting control device (110, 210-k) is configured to adjust duration of a mode change from the first lighting mode to the second lighting mode based at least in part on the lighting mode used by other luminaires (120-k, 220-k) that are next to the luminaire (120-k, 220-k) and/or within a predetermined distance from the luminaire (120-k, 220-k).

15. A lighting control device according to any preceding claim, wherein the lighting control device (110, 210-k) is further configured to control the light output from the luminaire (120-k, 220-k) to a user-selected light intensity, color and/or color temperature in accordance with one or more control signals received from a user-operable control panel or device.

16. A lighting control device according to claim 15, wherein the lighting control device (110, 210-k) is configured to redefine the duration the first lighting mode is used, light intensity level produced by the luminaire (120-k, 220-k) in a certain lighting mode, color produced by the luminaire (120-k, 220-k) in a certain lighting mode and/or color temperature produced by the luminaire (120-k, 220-k) in a certain lighting mode based at least in part on receiving control signals from a user-operable control panel or device repeatedly on different days.

17. A lighting control device according to any preceding claim, wherein the lighting control device is arranged in connection with the luminaire and configured to control that specific luminaire or the lighting control device is arranged to a control unit of the lighting system and configured to control multiple luminaires.

18. A method for controlling light output from at least one luminaire (120-k, 220-k), the method comprising:
controlling light output from the at least one luminaire (120-k, 220-k) in accordance with a lighting control logic, the method **characterized by**:
adjusting the lighting control logic at least in part on the information received from an occupancy sensor (132, 232-k) which senses motion and/or presence of a person in a certain area, wherein the adjustment comprises:
- receiving time information
- determining an average switch-on time for a day based on luminaire data collected from multiple days by taking into account the collected data about a time the occupancy sensor starts to sense motion on an area of a specific luminaire or a group of luminaires ,
- determining an average duration of a day based on a duration between the time of day the occupancy information starts to indicate presence of a person and the time of day on which the occupancy information no longer indicates presence of a person on that day and
selectingbetween the first lighting mode and second lighting mode to be used at a certain time interval wherein the selection is based at least in part on the determined average duration of the day and/or the determined average switch-on time, wherein at least one of the following is different in the first lighting mode than in the second lighting mode: the light intensity level produced by the luminaire (120-k, 220-k), color temperature of the light output by the luminaire (120-k, 220-k), color of the light output by the luminaire (120-k, 220-k).

19. A computer program for controlling light output from a luminaire, the computer program comprising computer readable program code configured to cause a lighting control device according to claim 1 to perform at least the method according to claim 18 when said program code is executed on the lighting control device.

## Patentansprüche

1. Beleuchtungssteuerungsvorrichtung zum Steuern von Lichtausgabe von mindestens einer Leuchte (120-k, 220-k), wobei die Beleuchtungssteuerungsvorrichtung (110, 210-k) Folgendes umfasst:
eine Beleuchtungssteuerungslogik zum Definieren der Weise des Steuerns von Lichtausgabe von der mindestens einen Leuchte (120-k, 220-k), wobei die Beleuchtungssteuerungsvorrichtung **gekennzeichnet ist durch**:
ein Anpassungsmittel zum Anpassen der Beleuchtungssteuerungslogik zumindest teilweise auf der Grundlage von Belegungsinformationen, erhalten von einem Belegungssensor (132, 232-k), ausgelegt zum Erfassen von Bewegung und/oder Anwesenheit einer Person in einem gewissen Gebiet, wobei das Anpassungsmittel ausgelegt ist zum:
- Empfangen von Zeitinformationen,
- Bestimmen einer durchschnittlichen Einschaltzeit für einen Tag auf der Grundlage von Leuchtendaten, gesammelt von mehreren Tagen **durch** Berücksichtigen der gesammelten Daten über eine Zeit, zu der der Belegungssensor beginnt, Bewegung in einem Gebiet einer spezifischen Leuchte oder einer Gruppe von Leuchten zu erfassen, und
- Bestimmen einer durchschnittlichen Dauer eines Tages auf der Grundlage einer Dauer zwischen der Tageszeit, zu der die Belegungsinformationen beginnen, Anwesenheit einer Person anzugeben, und der Tageszeit, zu der die Belegungsinformationen nicht mehr die Anwesenheit einer Person an dem Tag angeben, und
wobei die Beleuchtungssteuerungsvorrichtung ausgelegt ist zum Auswählen zwischen einem ersten Beleuchtungsmodus und einem zweiten Beleuchtungsmodus, der in einem gewissen Zeitintervall verwendet werden soll, wobei die Auswahl zumindest teilweise auf der bestimmten durchschnittlichen Dauer des Tages und/oder der durchschnittlichen Einschaltzeit basiert, wobei mindestens eines der Folgenden in dem ersten Beleuchtungsmodus anders als in dem zweiten Beleuchtungsmodus ist: der Lichtintensitätspegel, der durch die Leuchte (120-k, 220-k) produziert wird, die Farbe der Lichtausgabe **durch** die Leuchte (120-k, 220-k), die Farbtemperatur der Lichtausgabe **durch** die Leuchte (120-k, 220-k).

2. Beleuchtungssteuerungsvorrichtung nach Anspruch 1, wobei die Beleuchtungssteuerungsvorrichtung (110, 210-k) ausgelegt ist zum Aktivieren des ersten Beleuchtungsmodus zu der oder vor der bestimmten durchschnittlichen Einschaltzeit und/oder zum Wechseln aus dem ersten Beleuchtungsmodus in den zweiten Beleuchtungsmodus nach einer vordefinierten Dauer von der bestimmten durchschnittlichen Einschaltzeit, wobei die vordefinierte Dauer zumindest teilweise auf der bestimmten durchschnittlichen Dauer des Tages basiert, wobei die vordefinierte Dauer kürzer als die oder gleich der bestimmte(n) durchschnittliche(n) Dauer des Tages ist.

3. Beleuchtungssteuerungsvorrichtung nach Anspruch 1 oder 2, wobei die Beleuchtungssteuerungsvorrichtung (110, 210-k) ausgelegt ist zum Neudefinieren der durchschnittlichen Einschaltzeit und/oder der durchschnittlichen Dauer des Tages auf der Grundlage der Belegungsinformationen, wenn es eine Abweichung zwischen der bestimmten durchschnittlichen Einschaltzeit und/oder der bestimmten durchschnittlichen Dauer des Tages und Belegungsinformationen gibt.

4. Beleuchtungssteuerungsvorrichtung nach einem vorhergehenden Anspruch, wobei die Lichtintensität, die durch die Leuchte (120-k, 220-k) produziert wird, in dem ersten Beleuchtungsmodus höher als in dem zweiten Beleuchtungsmodus ist und/oder die Farbtemperatur, die durch die Leuchte (120-k, 220-k) produziert wird, in dem ersten Beleuchtungsmodus kühler als in dem zweiten Beleuchtungsmodus ist.

5. Beleuchtungssteuerungsvorrichtung nach einem vorhergehenden Anspruch, wobei die Beleuchtungssteuerungsvorrichtung einen dritten Beleuchtungsmodus aufweist und/oder die Beleuchtungssteuerungsvorrichtung ausgelegt ist zum Auswählen, wann der dritte Beleuchtungsmodus verwendet wird, zumindest teilweise auf der Grundlage von der bestimmten durchschnittlichen Dauer des Tages und/oder der bestimmten durchschnittlichen Einschaltzeit.

6. Beleuchtungssteuerungsvorrichtung nach einem vorhergehenden Anspruch, wobei die Lichtintensität, die Farbe und/oder die Farbtemperatur, die durch die Leuchte (120-k, 220-k) in dem ersten Beleuchtungsmodus und in dem zweiten Beleuchtungsmodus produziert werden, vordefiniert und/oder in dem Speicher (114, 415) der Beleuchtungssteuerungsvorrichtung (110, 210-k) gespeichert sind.

7. Beleuchtungssteuerungsvorrichtung nach einem vorhergehenden Anspruch, wobei die Beleuchtungssteuerungsvorrichtung (110, 210-k) ausgelegt ist zum Empfangen von Zeitinformationen von einer Gateway-Einheit, einer Steuereinheit und/oder einer externen Vorrichtung, wie etwa einem Mobiltelefon oder einem Computer.

8. Beleuchtungssteuerungsvorrichtung nach einem vorhergehenden Anspruch, wobei die Beleuchtungssteuerungsvorrichtung (110, 210-k) ausgelegt ist zum Anpassen der Lichtausgabe von der Leuchte (220-k) auf der Grundlage der Belegungsinformationen, so dass Lichtquellen (121-1, 221-k) in einen passiven Zustand geschaltet werden, nach einer kontinuierlichen Periode einer vorbestimmten Dauer, während der die Belegungsinformationen nicht angeben, dass Personen in dem überwachten Gebiet anwesend sind, und
die Lichtquellen (121-1, 221-k) in einen aktiven Zustand geschaltet werden, nachdem Belegung einer Person nach einer Nichtbelegungsperiode angegeben wird, wobei die Beleuchtungssteuerungsvorrichtung in dem aktiven Zustand konfiguriert ist zum Auswählen des Beleuchtungsmodus der Leuchte auf der Grundlage davon, welcher Modus der aktuelle Beleuchtungsmodus ist.

9. Beleuchtungssteuerungsvorrichtung nach einem vorhergehenden Anspruch, wobei der Wechsel aus dem ersten Beleuchtungsmodus in den zweiten Beleuchtungsmodus dafür konfiguriert ist, eine gewisse Dauer anzudauern, während der die Lichtintensität, die Farbe und/oder die Farbtemperatur der Leuchte (120-k, 220-k) kontinuierlich, graduell und/oder in Stufen von den Pegeln des ersten Beleuchtungsmodus zu dem zweiten Beleuchtungsmodus gewechselt werden.

10. Beleuchtungssteuerungsvorrichtung nach Anspruch 9, wobei die Lichtintensität, die Farbe und/oder die Farbtemperatur der Leuchte (120-k, 220-k) in dem aktiven Zustand der Leuchte grundsätzlich konstant gehalten werden und die Lichtintensität, die Farbe und/oder die Farbtemperatur derart ausgelegt sind, dass sie von den Pegeln des ersten Beleuchtungsmodus zu dem zweiten Beleuchtungsmodus nur gewechselt werden können, nachdem sich die Leuchte im passiven Zustand befunden hat und wieder in den aktiven Zustand eintritt.

11. Beleuchtungssteuerungsvorrichtung nach Ansprüchen 9 oder 10, wobei die Dauer des Wechsels aus dem ersten Beleuchtungsmodus in den zweiten Beleuchtungsmodus dafür konfiguriert ist, zumindest teilweise auf der Grundlage von der durchschnittlichen Dauer des Tages und/oder Dauern und/oder Frequenz der passiven Zustände zwischen den aktiven Zuständen der Leuchte (120-k, 220-k) bestimmt zu werden.

12. Beleuchtungssteuerungsvorrichtung nach einem vorhergehenden Anspruch, wobei die Leuchte (120-k, 220-k) eine Kommunikationseinheit (123-1, 223-k) zur Kommunikation mit einer Beleuchtungssteuerungsvorrichtung (110) des Beleuchtungssystems und/oder eine Beleuchtungssteuerungsvorrichtung (210-k), die zum Steuern anderer Leuchten (120-k, 220-k) dient, umfasst, wobei die Kommunikationseinheit eingerichtet ist zum Empfangen von Statusangaben von der mindestens einen Beleuchtungssteuerungsvorrichtung (110, 210-k), wobei die Statusangaben mindestens den durch die Leuchte (120-k, 220-k) verwendeten Beleuchtungsmodus umfassen.

13. Beleuchtungssteuerungsvorrichtung nach einem vorhergehenden Anspruch, wobei die Beleuchtungssteuerungsvorrichtung (110, 210-k) ausgelegt ist zum Abgleichen der Lichtintensitätspegel, der Farbe und/oder der Farbtemperatur auf die Lichtintensitätspegel, die Farbe und/oder die Farbtemperatur der anderen Leuchten (120-k, 220-k), die der Leuchte (120-k, 220-k) am nächsten sind, und/oder sich innerhalb einer vorbestimmten Distanz von der Leuchte (120-k, 220-k) befinden.

14. Beleuchtungssteuerungsvorrichtung nach einem vorhergehenden Anspruch, wobei die Beleuchtungssteuerungsvorrichtung (110, 210-k) ausgelegt ist zum Anpassen der Dauer eines Moduswechsels aus dem ersten Beleuchtungsmodus in den zweiten Beleuchtungsmodus zumindest teilweise auf der Grundlage des Beleuchtungsmodus, der von anderen Leuchten (120-k, 220-k), die der Leuchte (120-k, 220-k) am nächsten sind und/oder sich innerhalb einer vorbestimmten Distanz von der Leuchte (120-k, 220-k) befinden, verwendet wird.

15. Beleuchtungssteuerungsvorrichtung nach einem vorhergehenden Anspruch, wobei die Beleuchtungssteuerungsvorrichtung (110, 210-k) ferner ausgelegt ist zum Steuern der Lichtausgabe von der Leuchte (120-k, 220-k) auf eine benutzerausgewählte Lichtintensität, Farbe und/oder Farbtemperatur gemäß einem oder mehreren Steuersignalen, die von einem benutzerbetreibbaren Steuerungspult oder einer benutzerbetreibbaren Steuerungsvorrichtung empfangen werden.

16. Beleuchtungssteuerungsvorrichtung nach Anspruch 15, wobei die Beleuchtungssteuerungsvorrichtung (110, 210-k) ausgelegt ist zum Neudefinieren der Dauer des verwendeten Beleuchtungsmodus, des durch die Leuchte (120-k, 220-k) in einem gewissen Beleuchtungsmodus produzierten Lichtintensitätspegels, der durch die Leuchte (120-k, 220-k) in einem gewissen Beleuchtungsmodus produzierten Farbe und/oder der durch die Leuchte (120-k, 220-k) in einem gewissen Beleuchtungsmodus produzierten Farbtemperatur zumindest teilweise auf der Grundlage von wiederholtem Empfangen von Steuersignalen von einem benutzerbetreibbaren Steuerungspult oder einer benutzerbetreibbaren Steuerungsvorrichtung an verschiedenen Tagen.

17. Beleuchtungssteuerungsvorrichtung nach einem vorhergehenden Anspruch, wobei die Beleuchtungssteuerungsvorrichtung in Verbindung mit der Leuchte angeordnet ist und konfiguriert ist zum Steuern der spezifischen Leuchte oder die Beleuchtungssteuerungsvorrichtung an einer Steuereinheit angeordnet ist und konfiguriert ist zum Steuern mehrerer Leuchten.

18. Verfahren zum Steuern von Lichtausgabe von mindestens einer Leuchte (120-k, 220-k), wobei das Verfahren umfasst:
Steuern von Lichtausgabe von der mindestens einen Leuchte (120-k, 220-k) gemäß einer Beleuchtungssteuerungslogik, wobei das Verfahren **gekennzeichnet ist durch**:
Anpassen der Beleuchtungssteuerungslogik zumindest teilweise aufgrund von Informationen, die von einem Belegungssensor (132, 232-k), der Bewegung und/oder Anwesenheit einer Person in einem gewissen Gebiet erfasst, empfangen werden, wobei das Anpassen umfasst:
- Empfangen von Zeitinformationen
- Bestimmen einer durchschnittlichen Einschaltzeit für einen Tag auf der Grundlage von Leuchtendaten, gesammelt von mehreren Tagen **durch** Berücksichtigen der gesammelten Daten über eine Zeit, zu der der Belegungssensor beginnt, Bewegung in einem Gebiet einer spezifischen Leuchte oder einer Gruppe von Leuchten zu erfassen,
- Bestimmen einer durchschnittlichen Dauer eines Tages auf der Grundlage der Dauer zwischen der Tageszeit, zu der die Belegungsinformationen beginnen, Anwesenheit einer Person anzugeben, und der Tageszeit, zu der die Belegungsinformationen nicht mehr die Anwesenheit einer Person an dem Tag angeben, und
Auswählen zwischen dem ersten Beleuchtungsmodus und dem zweiten Beleuchtungsmodus, der in einem gewissen Zeitintervall verwendet werden soll, wobei die Auswahl zumindest teilweise auf der bestimmten durchschnittlichen Dauer des Tages und/oder der durchschnittlichen Einschaltzeit basiert, wobei mindestens eines der Folgenden in dem ersten Beleuchtungsmodus anders als in dem zweiten Beleuchtungsmodus ist: der Lichtintensitätspegel, der **durch** die Leuchte (120-k, 220-k) produziert wird, die Farbtemperatur der Lichtausgabe **durch** die Leuchte (120-k, 220-k), die Farbe der Lichtausgabe **durch** die Leuchte (120-k, 220-k).

19. Computerprogramm zum Steuern von Lichtausgabe von einer Leuchte, wobei das Computerprogramm computerlesbaren Programmcode umfasst, der dafür ausgelegt ist, eine Beleuchtungssteuerungsvorrichtung nach Anspruch 1 zu veranlassen, zumindest das Verfahren nach Anspruch 18 durchzuführen, wenn der Programmcode auf der Beleuchtungssteuerungsvorrichtung ausgeführt wird.

## Revendications

1. Dispositif de commande d'éclairage pour commander la lumière émise en sortie depuis au moins un luminaire (120-k, 220-k), le dispositif de commande d'éclairage (110, 210-k) comprenant :
une logique de commande d'éclairage pour définir la manière de commander la lumière émise en sortie depuis l'au moins un luminaire (120-k, 220-k), le dispositif de commande d'éclairage étant **caractérisé par** :
un moyen d'adaptation pour régler la logique de commande d'éclairage au moins en partie sur la base d'une information d'occupation reçue depuis un capteur d'occupation (132, 232-k) configuré pour détecter le mouvement et/ou la présence d'une personne dans une certaine zone, dans lequel le moyen d'adaptation est configuré pour :
- recevoir une information temporelle,
- déterminer un temps moyen de commutation dans l'état activé sur une journée sur la base de données de luminaire collectées sur de multiples journées en prenant en compte les données collectées un moment avant que le capteur d'occupation ne commence à détecter le mouvement sur une zone d'un luminaire spécifique ou d'un groupe de luminaires, et
- déterminer une durée moyenne d'une journée sur la base d'une durée entre l'instant de la journée auquel l'information d'occupation commence à indiquer la présence d'une personne et l'instant de la journée auquel l'information d'occupation n'indique plus la présence d'une personne sur cette journée et
dans lequel le dispositif de commande d'éclairage est configuré pour effectuer un choix entre un premier mode d'éclairage et un deuxième mode d'éclairage à utiliser selon un certain intervalle temporel, dans lequel le choix est basé au moins en partie sur la durée moyenne déterminée de la journée et/ou sur le temps moyen de commutation dans l'état activé déterminé, dans lequel au moins un élément informationnel de ceux qui suivent est différent dans le premier mode d'éclairage par comparaison avec le deuxième mode d'éclairage : le niveau de l'intensité lumineuse produite par le luminaire (120-k, 220-k), la couleur de la lumière émise en sortie par le luminaire (120-k, 220-k), la température de couleur de la lumière émise en sortie par le luminaire (120-k, 220-k).

2. Dispositif de commande d'éclairage selon la revendication 1, dans lequel le dispositif de commande d'éclairage (110, 210-k) est configuré pour activer le premier mode d'éclairage au temps moyen de commutation dans l'état activé déterminé ou avant celui-ci et/ou pour passer du premier mode d'éclairage au deuxième mode d'éclairage après une durée prédéfinie par rapport au temps moyen de commutation dans l'état activé déterminé, dans lequel la durée prédéfinie est basée au moins en partie sur la durée moyenne déterminée de la journée, et dans lequel la durée prédéfinie est inférieure ou égale à la durée moyenne déterminée de la journée.

3. Dispositif de commande d'éclairage selon la revendication 1 ou 2, dans lequel le dispositif de commande d'éclairage (110, 210-k) est configuré pour redéfinir le temps moyen de commutation dans l'état activé et/ou la durée moyenne de la journée sur la base de l'information d'occupation s'il y a un écart entre le temps moyen de commutation dans l'état activé déterminé et/ou la durée moyenne déterminée de la journée et l'information d'occupation.

4. Dispositif de commande d'éclairage selon l'une quelconque des revendications précédentes, dans lequel l'intensité lumineuse produite par le luminaire (120-k, 220-k) est plus élevée dans le premier mode d'éclairage que dans le deuxième mode d'éclairage, et/ou la température de couleur produite par le luminaire (120-k, 220-k) est plus froide dans le premier mode d'éclairage que dans le deuxième mode d'éclairage.

5. Dispositif de commande d'éclairage selon l'une quelconque des revendications précédentes, dans lequel le dispositif de commande d'éclairage comprend un troisième mode d'éclairage et/ou le dispositif de commande d'éclairage est configuré pour choisir lorsque le troisième mode d'éclairage est utilisé sur la base au moins en partie de la durée moyenne déterminée de la journée et/ou du temps moyen de commutation dans l'état activé déterminé.

6. Dispositif de commande d'éclairage selon l'une quelconque des revendications précédentes, dans lequel l'intensité lumineuse, la couleur et/ou la température de couleur produites par le luminaire (120-k, 220-k) dans le premier mode d'éclairage et dans le deuxième mode d'éclairage sont prédéfinies et/ou stockées dans la mémoire (114, 415) du dispositif de commande d'éclairage (110, 210-k).

7. Dispositif de commande d'éclairage selon l'une quelconque des revendications précédentes, dans lequel le dispositif de commande d'éclairage (110, 210-k) est configuré pour recevoir l'information temporelle en provenance d'une unité de passerelle, d'une unité de commande et/ou d'un dispositif externe tel qu'un téléphone mobile ou un ordinateur.

8. Dispositif de commande d'éclairage selon l'une quelconque des revendications précédentes, dans lequel le dispositif de commande d'éclairage (110, 210-k) est configuré pour régler la lumière émise en sortie depuis le luminaire (220-k) sur la base de l'information d'occupation de telle sorte que des sources de lumière (121-1, 221-k) soient commutées dans l'état passif après une période continue d'une durée prédéterminée pendant laquelle l'information d'occupation n'indique pas des personnes présentes dans la zone surveillée, et
les sources de lumière (121-1, 221-k) sont commutées dans un état actif après que l'occupation d'une personne est indiquée après une période de non occupation, dans lequel, dans l'état actif, le dispositif de commande d'éclairage est configuré pour choisir le mode d'éclairage du luminaire sur la base du mode qui est le mode d'éclairage courant.

9. Dispositif de commande d'éclairage selon l'une quelconque des revendications précédentes, dans lequel le passage du premier mode d'éclairage au deuxième mode d'éclairage est configuré pour s'étendre sur une certaine durée pendant laquelle l'intensité lumineuse, la couleur et/ou la température de couleur du luminaire (120-k, 220-k) sont passées des niveaux du premier mode d'éclairage aux niveaux du deuxième mode d'éclairage en continu, de façon progressive et/ou par pas.

10. Dispositif de commande d'éclairage selon la revendication 9, dans lequel l'intensité lumineuse, la couleur et/ou la température de couleur du luminaire (120-k, 220-k) sont maintenues essentiellement constantes dans l'état actif du luminaire, et l'intensité lumineuse, la couleur et/ou la température de couleur sont configurées pour être passées des niveaux du premier mode d'éclairage aux niveaux du deuxième mode d'éclairage seulement après que le luminaire a été dans l'état passif et qu'il entre à nouveau dans l'état actif.

11. Dispositif de commande d'éclairage selon les revendications 9 ou 10, dans lequel la durée du passage du premier mode d'éclairage au deuxième mode d'éclairage est configurée pour être déterminée sur la base au moins en partie de la durée moyenne de la journée et/ou de durées et/ou de fréquence des états passifs entre les états actifs du luminaire (120-k, 220-k).

12. Dispositif de commande d'éclairage selon l'une quelconque des revendications précédentes, dans lequel le luminaire (120-k, 220-k) comprend une unité de communication (123-1, 223-k) pour une communication avec un dispositif de commande d'éclairage (110) du système d'éclairage et/ou avec des dispositifs de commande d'éclairage (210-k) qui servent à commander d'autres luminaires (120-k, 220-k), l'unité de communication étant agencée pour recevoir des indications d'état en provenance de l'au moins un dispositif de commande d'éclairage (110, 210-k), les indications d'état comprenant au moins le mode d'éclairage utilisé par le luminaire (120-k, 220-k).

13. Dispositif de commande d'éclairage selon l'une quelconque des revendications précédentes, dans lequel le dispositif de commande d'éclairage (110, 210-k) est configuré pour faire correspondre les niveaux d'intensité lumineuse, la couleur et/ou la température de couleur aux niveaux d'intensité lumineuse, à la couleur et/ou à la température de couleur des autres luminaires (120-k, 220-k) qui sont proches du luminaire (120-k, 220-k) et/ou à l'intérieur d'une distance prédéterminée par rapport au luminaire (120-k, 220-k).

14. Dispositif de commande d'éclairage selon l'une quelconque des revendications précédentes, dans lequel le dispositif de commande d'éclairage (110, 210-k) est configuré pour régler la durée d'un changement de mode depuis le premier mode d'éclairage selon le deuxième mode d'éclairage sur la base au moins en partie du mode d'éclairage utilisé par d'autres luminaires (120-k, 220-k) qui sont proches du luminaire (120-k, 220-k) et/ou à l'intérieur d'une distance prédéterminée par rapport au luminaire (120-k, 220-k).

15. Dispositif de commande d'éclairage selon l'une quelconque des revendications précédentes, dans lequel le dispositif de commande d'éclairage (110, 210-k) est en outre configuré pour commander la lumière émise en sortie depuis le luminaire (120-k, 220-k) selon une intensité lumineuse, une couleur et/ou une température de couleur choisies par utilisateur conformément à un signal de commande ou à plusieurs signaux de commande reçu(s) depuis un panneau ou dispositif de commande actionnable par utilisateur.

16. Dispositif de commande d'éclairage selon la revendication 15, dans lequel le dispositif de commande d'éclairage (110, 210-k) est configuré pour redéfinir la durée pendant laquelle le premier mode d'éclairage est utilisé, le niveau de l'intensité lumineuse produite par le luminaire (120-k, 220-k) dans un certain mode d'éclairage, la couleur produite par le luminaire (120-k, 220-k) dans un certain mode d'éclairage et/ou la température de couleur produite par le luminaire (120-k, 220-k) dans un certain mode d'éclairage sur la base au moins en partie de la réception de signaux de commande en provenance d'un panneau ou dispositif de commande actionnable par utilisateur de façon répétée sur différentes journées.

17. Dispositif de commande d'éclairage selon l'une quelconque des revendications précédentes, dans lequel le dispositif de commande d'éclairage est agencé en connexion avec le luminaire et est configuré pour commander ce luminaire spécifique ou le dispositif de commande d'éclairage est agencé sur une unité de commande du système d'éclairage et est configuré pour commander de multiples luminaires.

18. Procédé pour commander la lumière émise en sortie depuis au moins un luminaire (120-k, 220-k), le procédé comprenant :
la commande de la lumière émise en sortie depuis l'au moins un luminaire (120-k, 220-k) conformément à une logique de commande d'éclairage, le procédé étant **caractérisé par** :
le réglage de la logique de commande d'éclairage au moins en partie sur la base de l'information reçue depuis un capteur d'occupation (132, 232-k) qui détecte le mouvement et/ou la présence d'une personne dans une certaine zone, dans lequel le réglage comprend :
- la réception d'une information temporelle,
- la détermination d'un temps moyen de commutation dans l'état activé sur une journée sur la base de données de luminaire collectées sur de multiples journées en prenant en compte les données collectées un moment avant que le capteur d'occupation ne commence à détecter le mouvement sur une zone d'un luminaire spécifique ou d'un groupe de luminaires, et
- la détermination d'une durée moyenne d'une journée sur la base d'une durée entre l'instant de la journée auquel l'information d'occupation commence à indiquer la présence d'une personne et l'instant de la journée auquel l'information d'occupation n'indique plus la présence d'une personne sur cette journée et
la réalisation d'un choix entre un premier mode d'éclairage et un deuxième mode d'éclairage à utiliser selon un certain intervalle temporel, dans lequel le choix est basé au moins en partie sur la durée moyenne déterminée de la journée et/ou sur le temps moyen de commutation dans l'état activé déterminé, dans lequel au moins un élément informationnel de ceux qui suivent est différent dans le premier mode d'éclairage par comparaison avec le deuxième mode d'éclairage : le niveau de l'intensité lumineuse produite par le luminaire (120-k, 220-k), la température de couleur de la lumière émise en sortie par le luminaire (120-k, 220-k), la couleur de la lumière émise en sortie par le luminaire (120-k, 220-k) .

19. Programme informatique pour commander la lumière émise en sortie depuis un luminaire, le programme informatique comprenant un code de programme lisible par ordinateur configuré pour forcer un dispositif de commande d'éclairage selon la revendication 1 à réaliser au moins le procédé selon la revendication 18 lorsque ledit code de programme est exécuté sur le dispositif de commande d'éclairage.
